# EUROPEAN PATENT APPLICATION

(11) **EP 1 944 368 A1**
(43) Date of publication of application: **16.07.2008**
(21) Application number: 08150067.0
(22) Date of filing: 07.01.2008
(51) Int. Cl.: C12N 15/10

(54) **Nucleic acid isolation method by heating on magnetic support**

(30) Priority: 15.01.2007 JP 2007006409
(71) Applicant: Konica Minolta Medical & Graphic, Inc., Hino-shi, Tokyo 191-8511 (JP)
(72) Inventor: Miyazaki, Koji, Konica Minolta Medical & Graphic, Inc., Tokyo 191-8511 (JP); Nakajima, Akihisa, Konica Minolta Tech. Center, Inc., Tokyo 191-8511 (JP); Usui, Kanako, Takara Bio Ink, Shiga 520-2193 (JP); Hino, Fumitsugu, Takara Bio Ink, Shiga 520-2193 (JP); Mukai, Hiroyuki, Takara Bio Ink, Shiga 520-2193 (JP); Kato, Ikunoshin, Takara Bio Ink, Shiga 520-2193 (JP)
(74) Representative: Henkel, Feiler & Hänzel

(57) **Abstract**

A method for isolating a nucleic acid from a cell-containing sample by using a magnet with a removable cover attached thereto and a plurality of containers arranged on a table, the plurality of containers having (1) a first container for mixing a cell-containing sample and magnetic beads to which cells can be adhered, (2) a cleaning tank for cleaning the magnetic beads with cells adhering thereto, and (3) a second container for heating the cleaned magnetic beads, the method includes: mixing a liquid including cell-containing sample and magnetic beads in the cell-containing sample in the first container so as to adhere cells in the cell-containing sample to magnetic beads; taking out the magnetic beads from the first container and cleaning the magnetic beads; and suspending the magnetic beads in a re-suspension buffer in the second container and heating the second container at 80 to 120 °C for 20 to 300.

## Description

### CROSS REFERENCE TO RELATED APPLICATION

The present application is based on Japanese Patent Application No. 2007-6409 filed with Japan Patent Office on January 15, 2007.

### BACKGROUND OF THE INVENTION FIELD OF THE INVENTION

The present invention relates a method for separating a nucleic acid from a sample including cells and more particularly to a method for destroying cells adhered to a magnetic support by heating and isolating a discharged nucleic acid and a kit and an apparatus for executing the method.

### DESCRIPTION OF THE RELATED ART

At present, the range of use of a nucleic acid as a sample or a material increases in the scientific research, medical field, and industrial circles and a method for extracting and isolating efficiently and collectably a nucleic acid from various samples is required.

As a method for obtaining a nucleic acid from a sample containing cells, the phenol-chloroform extraction method has been used from long ago. This classic method modifies, dissolves, or precipitates a water-nonsoluble specimen component such as protein substance or lipid using phenol-chloroform and on the other hand, uses the difference in the solubility for dissolving a nucleic acid in the water phase. As an alternative method not using such a poisonous solvent, various methods have been proposed in recent years.

A method for obtaining a bacteria nucleic acid by exposing heat effective in bacteriolysis to mycobacteria in place of using a bacteriolytic agent and other conditions such as mechanical methods for bacteriolysis is disclosed (refer to U.S. Pat. No. 5,376,527). As dissolution of cells by heating, an aqueous sample of distal blood single nucleic cells may be used (refer to U.S. Pat. No. 5,334,499).

A nucleic acid isolation method for binding cells to a magnetic support, acting a surface active agent and/or a chaotorope reagent, thereby binding a liberated nucleic acid to the same magnetic support is proposed (refer to International Patent Publication No. WO 98/51693). Further, as a suitable method for automating isolation of a nucleic acid, a method for binding a sample to a solid support, applying a solution of cells (Gentra Systems, Ltd.) to it, thereby liberating a nucleic acid, and insolating it is disclosed (refer to International Patent Publication No. WO 99/13976). In this method, to promote elusion of a nucleic acid from the solid support, the heating step is included supplementarily

Further, a method for liberating a nucleic acid by crushing and grinding by the crushing dispersion action (based on the ultrasonic vibration and vertical motion) of a processing member with a nucleic acid containing sample coated, binding the nucleic acid to a magnetic carrier which is a nucleic acid extraction carrier, and extracting and separating efficiently the nucleic acid using the magnetized processing member, magnetic carrier, and magnetic action is known (refer to Japanese Patent Application Publication No. 2004-337137). As a cleaning liquid, use of a chaotopic substance or ethanol is described, though the nucleic acid extraction solution is not disclosed in detail.

As mentioned above, when the nucleic acid extracted using an organic solvent, a chaotorope reagent, a bacteriolytic agent, or a surface active agent is used as a mold of the DNA amplification method of polymerase chain reaction, SDA (strand displacement amplification), LCR (ligase chain reaction), gap LCR, ICAN (isothermal and chimeric primer-initiated amplification of nucleic acids), LAMP (loop-mediated isothermal amplification), TMA (transcription-mediated amplification), Qβ replicase amplification, TAS (transcription amplification system), 3SR (self-sustained sequence replication system), or NASBA (nucleic acid sequence-based amplification or is digested by a limiting enzyme, such drugs remaining in the nucleic acid solution cause obstacles and the removing process thereof is troublesome most. Particularly, to a very small quantity of sample, such an extraction method cannot respond.

In U.S. Pat. Nos. 5,763,185 and 6,210,881 the eliminating method of a nucleic acid hybridization inhibitor is recorded. Namely, the method comprises a step of permitting an action substance for solving the inhibitor and preventing the nucleic acid from releasing from cells to make contact with the concerned cells and a step of separating the concerned cells from the action substance by centrifugation.

As mentioned above, a method for extracting and refining briefly and quickly a nucleic acid with high purity from various nucleic acid containing samples of the organism origin without using a poisonous solvent or a corrosive reagent and expecting automation is not available at present and is desired earnestly.

In relation to it, in U.S. Pat. No. 5,554,503, a method for centrifuging (4000 x G at minimum, 5 minutes at least) saliva, cleaning and centrifuging (12000 x G at least) pellets, then heating at 95 to 120 °C for 5 to 30 minutes, destroying cell membranes, and isolating a nucleic acid is proposed. Furthermore, in International Patent Publication No. WO 01/053525, a nucleic acid isolation method for binding cells to a non-peculiar ligand on a solid support, dissolving bound cells, and binding a discharged nucleic acid to the solid support is recorded.

### SUMMARY

The inventors pursued earnest studies with this foregoing in view and found that cells in a sample are bound to a magnetic support, and the cell membranes are destroyed by the heating process, thus a nucleic acid can be obtained, thereby could accomplish the present invention. On the basis of the method of the present invention, a kit for separating a nucleic acid from a specimen briefly with high purity is provided.

According to one aspect of the present invention, there is provided a method for isolating a nucleic acid from a cell-containing sample by using a magnet with a removable cover attached thereto and a plurality of containers arranged on a table, the plurality of containers comprising (1) a first container for mixing a cell-containing sample and magnetic beads to which cells can be adhered, (2) a cleaning tank for cleaning the magnetic beads with cells adhering thereto, and (3) a second container for heating the cleaned magnetic beads, the method comprises: mixing a liquid including cell-containing sample and magnetic beads in the cell-containing sample in the first container so as to adhere cells in the cell-containing sample to magnetic beads; taking out the magnetic beads with cells adhering thereto from the first container and cleaning the magnetic beads; and suspending the magnetic beads in a re-suspension buffer in the second container and heating the second container at 80 to 120 °C for 20 to 300 seconds so as to isolate a nucleic acid from the cells, wherein all the processes are completed within 600 seconds.

Among before mixing the magnetic beads with the sample liquid, after mixing, and during cleaning the magnetic beads, at least at any stage, it is necessary to suspend the magnetic beads. Further, it is desirable to arrange the containers and cleaning tank in the order in which they are processed on a rectangular or circular table.

The aforementioned cells are desirably microbes belonging to chlamydia (chlamydia group), neisseria (Neisseria group), and mycobacteria (mycobacterium group).

The identifying method of the cell species characterized in identification of a nucleic acid obtained by any isolation method recorded above by the nucleic acid amplification method is included in the present invention.

In the present invention, furthermore, a kit characterized in that the magnetic beads, cleaning buffer, and re-suspension buffer for executing the aforementioned method are respectively charged beforehand in the containers is included. Furthermore, a nucleic acid isolation apparatus for executing the aforementioned method is included in the present invention.

The gene inspection method including the stage of amplifying and detecting a nucleic acid by an apparatus having a microchip for isolating a gene by the aforementioned method is also included in the present invention.

### BRIEF DESCRIPTION OF THE DRAWINGS

Fig. 1 shows the outline of the operation procedure of the method of the present invention.
Fig. 2 shows a conceptual diagram of the operation of the nucleic acid isolation kit using magnetic beads and a magnet with a cover. The contents of Steps (1) to (7) will be explained in the text.
Fig. 3 shows a typical example of each container of the nucleic acid isolation instrument using magnetic beads. φ indicates a diameter of the circle, and R indicates a radius of the circle, and SR indicates a radius of the sphere. R2 shown at the bottom of the container 1 indicates that the inner wall thereof is rounded in a circle with a diameter of 2 mm and R4 indicates that the outer wall thereof is rounded in a circle with a diameter of 4 mm. The same may be said with SR5 and 75 and SR 7 and 25 indicated at the bottom of the container 2.
Fig. 4 shows an aspect of the magnet cover of the nucleic acid isolation instrument. The meanings indicated by the symbols φ, R, and SR are the same as those shown in Fig. 3. SR4.1 and SR5.5 indicated at the bottom are the same as the explanation in Fig. 3. In the drawing (lower drawing) showing the state that the magnet cover is inserted into the container 1, 4.4 indicates the height of the liquid level when a sample of 1 mL is put in and 0.35 indicates the height of the liquid level rising when the magnet and magnet cover are inserted into the container 1.
Fig. 5(a) shows the outline of the horizontal movement apparatus of the nucleic acid isolation instrument and Fig. 5(b) shows the outline of the rotary movement apparatus.
Fig. 6 is a schematic diagram showing movement of the rotary movement apparatus shown in Fig. 5(b).
Fig. 7 is a schematic diagram of the microchip mounted in the nucleic acid analytical apparatus for executing the gene inspection method of the present invention.

### DETAILED DESCRIPTION OF THE PREFERRED EMBODIMENTS

### Nucleic acid isolation method

The method of the present invention is a method for isolating a nucleic acid from a cell-containing sample, which is characterized in that by using a magnet with a removable cover attached and the following containers arranged on one table:
(1) a container 1 for mixing a cell-containing sample and magnetic beads to which cells can be adhered,
(2) a cleaning tank for cleaning the magnetic beads with cells attached, and
(3) a container 2 for heating the cleaned magnetic beads,
the process of adhering and cleaning cells in the cell-containing sample to magnetic beads and isolating a nucleic acid from the cells includes:
a step of mixing the liquid and magnetic beads in the cell-containing sample in the container 1,
a step of taking out and cleaning the magnetic beads from the container 1, and then
a step of suspending the magnetic beads in a re-suspension buffer in the container 2 and heating the container 2 at 80 to 12 °C for 20 to 300 seconds, and
the overall process is completed within 600 seconds.

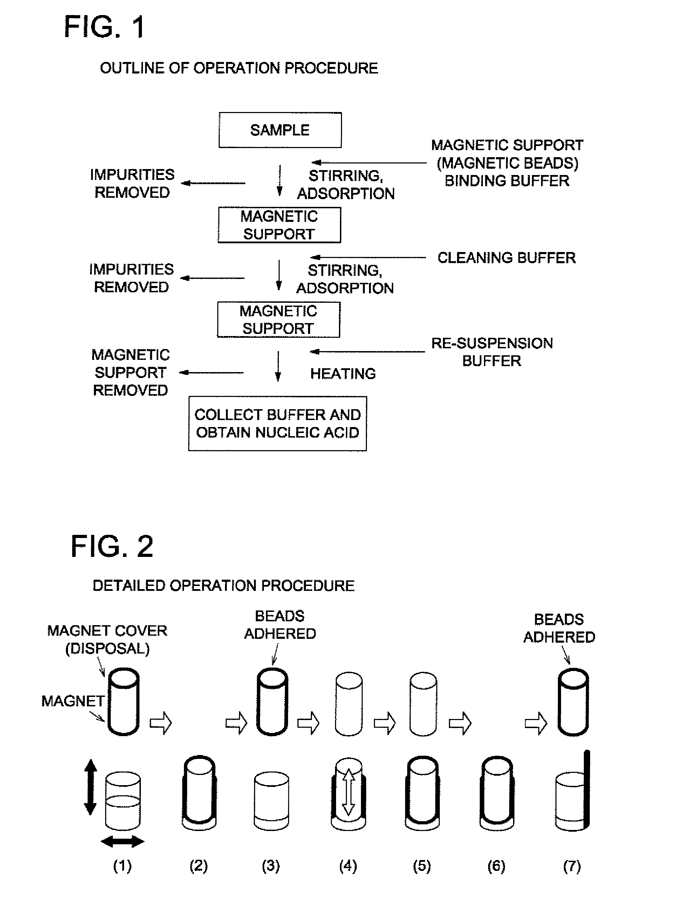

The method of the present invention is to adsorb, adhere, or bind (in this specification, in any case, it may be expressed just as "adherence") cells included in a sample liquid to a magnetic support, preferably magnetic beads, when the cells are separated from impurities in the sample liquid and then are bound to the magnetic support, destroy the cell membranes by heating so as to discharge a nucleic acid from the cells, thereby collect it. Regarding the operation procedure, the main point thereof is shown in Fig. 1.

The cell-containing sample and magnetic support (preferably magnetic beads) are mixed and stirred, and the cells in the sample liquid are adsorbed, adhered, or bound to the magnetic support. At that time, to promote adsorption, adhesion, or binding of the cells to the magnetic support, an appropriate binding buffer may be used. Generally, a sample is a fluid such as a liquid, though a solid sample is dissolved or dispersed in an appropriates solvent (for example, a binding buffer) .

When the magnetic support is separated (solid-liquid separation) from the sample, impurities and unnecessary articles included in the sample are separated and removed and the magnetic support with cells adhered is obtained.

Next, the operation of stirring and cleaning the magnetic support together with a cleaning buffer and then the solid-liquid separation operation are performed again, thus impurities adhered to the magnetic support are removed more. As mentioned above, the solid-liquid separation process is included in the method of the present invention, though the solid-liquid separation is executed by the magnetic action or centrifugal force action. Preferably, by acting the magnetism by an external magnet to the container including the magnetic support, the solid-liquid separation is executed. A more preferable aspect is that when moving the magnetic beads, the magnetic beads are operated by taking in and out a magnet with a removable cover attached and this is a point characterized in the method.

The magnetic support adsorbing, adhering, or binding cells is suspended in the re-suspension buffer and the cell membranes are destroyed by the heating process.

Then, the solid-liquid separation is executed by the aforementioned means, thus the magnetic support is separated from the re-suspension buffer including the nucleic acid liberated from the cells, and the buffer is collected, and the desired nucleic acid can be isolated from the buffer. The method does not include the operation such as the filtering and decompressing treatment, so that the nucleic acid can be isolated briefly and quickly. Further, the method is characterized in that among before mixing the magnetic beads with the sample liquid, after mixing, and during cleaning the magnetic beads, at least at any stage, the magnetic beads are suspended. The significance of such suspension is as indicated below. Before mixing, the magnetic beads are condensed during preservation due to the mutual magnetic force action of the magnetic beads. Therefore, when the magnetic beads are dispersed by suspension, at time of mixture with the sample liquid, the contact surface area and number of contact times between the magnetic beads and the cells are increased. When the suspension state is kept even after mixture with the sample liquid, the magnetic beads are dispersed by suspension in the sample liquid, and the contact surface area and number of contact times between the magnetic beads and the cells are increased. Further, during cleaning, the magnetic beads are dispersed by suspension in the cleaning buffer, and the contact surface area and number of contact times between the cleaning effective material of the cleaning buffer and the magnetic beads (cells are adhered on the surfaces thereof) are increased. By the effects thereof, improvement of the collectable quantity by separating cells and a nucleic acid from the sample liquid at each stage and purity thereof can be expected.

Hereinafter, the method of the present invention will be explained more in detail.

Cells taken up in the present invention may be any of microbes (bacteria, mold, yeast, etc.), cells of plants and animals, and cultures of cells and are not restricted particularly. Those cells are preferably cells of microbes and particularly are desirably microbes belonging to chlamydia (chlamydia group), neisseria (Neisseria group), and mycobacteria (mycobacterium group).

The sample is a sample containing the cells aforementioned and if it is a living body originated sample, there are no particular restrictions on it and for example, almost all living body originated samples such as whole blood, blood plasma, serum, buffy coat, urine, fecal matter, saliva, phlegm, cerebral spinal fluid, semen, tissue (for example, cancerous tissue, lymph node, etc.), and cell culture (for example, cell culture of manual, bacteria culture, etc.) conform to it. The nucleic acid containing sample includes a sample possible to mix or contain microbes and all samples (foods, biological formulations, etc.) possible to contain a nucleic acid. Or, an environmental sample such as soil or drain possible to contain living things may be cited. The form of samples is preferably a sample of a fluid and generally a solution or a liquid of a suspension. The sample may be a dissolvable solid or a solid floating in a liquid.

The nucleic acid to be isolated of the present invention is DNA or RNA, and DNA includes genome DNA and cDNA and RNA includes mRNA, tRNA, and rRNA. Furthermore, a single chain and two chains are no particular object. A preferable range of the DNA quantity to be isolated is 0.001 ng to 1 mg. In this specification, the "gene" is referred to as a nucleic acid carrying heredity information for revealing any function, that is, DNA or RNA, though it may be referred to as only a form of DNA or RNA which is a chemical substance. Further, the "base" is referred to as the nucleic acid base of nucleotide.

For the aforementioned destruction of cell membranes, various well-known physical methods may be used. Cell membrane destruction is executed preferably by heating. The reason is that the heating is simple and as mentioned above, there is no need to remove later the medicine used for the cell membrane destruction. Concretely, the heating aforementioned is heating within the temperature range in which the nucleic acid is not degenerated by heating, that is, at 70 to 120 °C, preferably at 80 to 120 °C, and more preferably at 80 to 100 °C for 20 seconds to 10 minutes, preferably for 20 seconds to 300 seconds. The heating conditions (temperature, time) vary with the kind of cells or bacteria (size, composition and thickness of cell membranes), so that the heating conditions may be selected appropriately within the aforementioned range. The heating is executed by various appropriate heating means, though a dry heat block, a hot water bath, a microwave oven, and various heaters may be cited. However, the heat means are not limited to them.

Further, in addition to the steps aforementioned, a step of condensing a nucleic acid liberated by vaporizing water by heating may be included. The heating to be applied is within the temperature range in which the nucleic acid is not regenerated by heating. The cell membrane destruction aforementioned is executed by heating, so that the cell membrane destruction step by heating can serve as the condensation step.

### - Magnetic support

According to the present invention, the magnetic support used to adhere target cells is a water-insoluble carrier. The material for forming the water-insoluble magnetic support may be water-insoluble. Here, water-insoluble, concretely, means a solid phase not dissolving in water and a water solution including any other water soluble composition. The solid support is used widely at present for fixing and separation and either of the proposed well-known support and matrix is acceptable.

Concretely, an inorganic compound, a metal, a metallic oxide, an organic compound, and a composite material composed of a combination thereof are included. Cells contained in a sample are adhered or bound to the magnetic support, though the magnetic support, if it adsorbs, adheres, or binds cells, is not restricted particularly on the material, shape, and size. Preferably, it may be made of a material providing a large surface area to bind cells, that is, to bind a nucleic acid.

The material used concretely as a magnetic support is not restricted particularly, though it may be generally a synthetic high polymeric organic substance such as polystyrene, polypropylene, polyacrylate, polymethyl methacrylate, polyethylene, polyamide, or latex, or an inorganic substance such as glass, silica, silicon dioxide, silicon nitride, zirconium oxide, aluminum oxide, sodium oxide, calcium oxide, magnesium oxide, or zinc oxide, or a metal such as stainless steel or zirconium. Among them, glass, silica, latex, and a polymer material are preferable and among them, an organic polymer, particularly polystyrene is preferable. These materials have generally an irregular surface and may be, for example, porous or granular, for example, particles, fibers, a web, a sinter, or a sieve.

Therefore, the shape of the magnetic support used in the method of the present invention is not restricted particularly, though the granular shape, cylindrical shape, laminar shape, a sheet, a gel, a membrane, a fiber shape, a capillary, a strip, and a filter may be cited, and it is preferably granular. A granular material, for example, beads are generally preferable because the binding ability thereof is high and polymer beads are preferable particularly,

As a granular form, for example, the spherical shape, ellipsoid shape, cone shape, cubic shape, and rectangular parallelepiped shape may be considered. Among them, a carrier of a spherical particle is manufactured easily and is preferable in view that when in use, the magnetic support can be rotated and stirred easily. The size of beads as a magnetic support for adhering or binding cells is desired to be 0.5 to 10 µm, preferably 2 to 6 µm. When the average particle diameter is smaller than 0.5 µm, if the concerned bead bodies contain a magnetic substance, they do not reveal a sufficient magnetic response, and a considerably long period of time is taken to separate the concerned particles, and a considerably large magnetic force is required to separate them. On the other hand, when the particle diameter is larger than 10 µm, the concerned particles precipitate easily in an aqueous medium, so that when trapping cells, the operation of stirring the medium is required. Further, the surface area of each particle body is reduced, so that it may be difficult to trap a sufficient amount of cells.

In addition to the case that all the beads including the surfaces are composed of the same material, a hybrid substance which may be composed of a plurality of materials whenever necessary may be used. For example, it can respond to automation of analysis, so that composite beads may be cited that the core portion is made of a magnetic response material such as iron oxide or chromium oxide and the surface thereof is covered with an organic synthesis polymer.

In the respect that the magnetic support binding cells can be separated (solid and liquid separation) easily from the sample liquid by the magnetic force of the magnet and particles can be collected, it is preferable that the magnetic support contains a magnetic substance such as a paramegnetic substance, a ferroparamagnetic substance, or a ferromagnetic substance and it is more preferable that the magnetic support contains both or either of the paramegnetic substance and ferroparamagnetic substance. Particularly, in the respect that there is no or little residual magnetization, it is preferable to use the ferroparamagnetic substance.

As a concrete example of such a magnetic substance, a metal such as iron oxide (Fe₃O₄), γ-iron oxide (γ-Fe₂O₃), various types of ferrite, iron, manganese, cobalt, or chromium, and various alloys of cobalt, nickel, and manganese may be cited. Among them, iron oxide (Fe₃O₄) is preferable particularly.

The magnetic substance used in the present invention is beads composed of particles with a small particle diameter and it is preferable that the magnetic substance has an excellent magnetism separation property (performance to separate beads by the magnetism in a short period of time) and can be re-dispersed by the slow vertical shaking operation.

The magnetic substance containing rate in the magnetic beads, since the containing rate of non-magnetic organic substances is 30 wt% or more, is assumed as 70 wt% or less, though preferably 20 to 70 wt%, more preferably 30 to 70 wt%. When the rate is less than 20 wt%, a sufficient magnetic response is not revealed and it may be difficult to separate particles in a short period of time by a necessary magnetic force. On the other hand, when the rate is more than 70 wt%, the amount of a magnetic substance exposed on the surfaces of the particle bodies is increased, so that the constituent component of the concerned magnetic substance, for example, iron ions are eluted, thus other materials may be adversely affected when in use, and the particle bodies are made fragile, thus practical strength cannot be obtained.

By this method of the present invention, when the sample liquid including cells and magnetic support (preferably magnetic beads) are mixed and cells are adhered, bound, or adsorbed to the magnetic support by this mixture, cells can be accumulated efficiently on the surface thereof. Even when cells are not adhered to the magnetic support, cells can be accumulated by the magnetic force or centrifugal force. Therefore, it is desirable that cells are adsorbed by the magnetic support, though cells may not be adhered.

Cells, particularly bacterium cells may not be adhered to the magnetic support. Furthermore, to promote surely adsorption and adhesion of cells, to the surface of the magnetic support, the reactive functional group such as the group having an affinity to cells, the amino group, oxy-carbonylimidazole group, or N-hydroxysuccinic acid imide group, or a "functional substance" such as sugar showing peculiarly an affinity to the target cells, a sugar protein substance, an antibody, lectin, or a cell adhesion factor may be bound or suitable coating for modification and binding of the surface structure thereof may be executed.

Depending on the sample, when the concentration of cells contained in it, particularly cells of the target bacteria is low, the operations of processing a large amount of sample solution and separating and condensing it are necessary. By the method of the present invention for binding or adhering cells to the magnetic support and extracting simply a nucleic acid in the cells, by a simple operation, such a sample process can be performed quickly. Particularly, the solid-liquid separation process of the present invention using magnetic beads and a magnet with a removable cover attached is convenient extremely even when there is only a small amount of sample available. In such a case, in the process of separation and extraction, a loss is caused to cells or a nucleic acid and there is a case that the target final collection amount of a nucleic acid may be smaller than a suitable amount for the subsequent analysis, while by the method of the present invention, little loss is caused during such isolation. The method of the present invention does not use a chaotorope reagent, a surface active agent, or a medicine such as a bacteriolytic agent for adversely affecting the nucleic acid amplification reaction, hybridization, limiting enzyme reaction, detection reaction, and electrophoresis analysis which are provided at the subsequent steps, so that the isolated nucleic acid can be applied straight to the amplification reaction. Therefore, even if the sample quantity is very small, by the method of the present invention, from cells, at a high yield, a nucleic acid with high purity can be isolated.

A method for identifying the kind of bacteria cells using the nucleic acid obtained by the separation method aforementioned is included in the present invention. Concretely, the nucleic acid extracted and isolated from the bacteria cells included in the sample is amplified by the DNA amplification method such as PCR, SDA, LCR, ICAN, LAMP, TMA, TAS, 3SR, or NASBA, and analysis of the amplified nucleic, for example, the base arrangement decision, hybridization method, and the Southern blot analysis are executed, and the results are compared with the standard or comparison base arrangement, thus the kind of bacteria cells can be identified.

Kit: The kit relating to the present invention is a kit for executing the nucleic acid isolation method aforementioned. Namely, the concerned kit is a set of components necessary to execute the method of the present invention, concretely including various reagents, magnetic support (beads), and nucleic acid isolation instruments. Among the reagents, a dissolution (or dilution) liquid for dissolving (diluting) a sample, a cleaning liquid, and various buffer solutions are included. A kit in an aspect that magnetic beads, a binding buffer, a cleaning buffer (cleaning liquid), and a re-suspension buffer (re-suspension liquid) are respectively charged beforehand in containers is desirable. In the set of necessary components, an exclusive instrument (that is, nucleic acid isolation instrument) for adhering or binding furthermore cells to the magnetic support, destroying cell membranes thereof, and extracting a nucleic acid contained internally is included as a kit element. Using these nucleic acid isolation instruments, the nucleic acid isolation method of the present invention aforementioned can be executed.

To execute the method of the present invention, tools or apparatuses other than the aforementioned may be required, though they are included properly as components of the kit of the present invention. For solid-liquid separation, the centrifugal force may be used, though in such a case, a small centrifuge is used.

In the nucleic acid isolation method of the present invention, at least two kinds of buffer solutions are used. The binding buffer is composed of salts and alcohol, and as salts, 0.75M ammonium acetate (NH₄Ac), sodium chloride, potassium chloride, sodium acetate, and potassium acetate may be cited, and as alcohol, isopropanol, ethanol, methanol, and n-butanol may be cited. The cleaning buffer may use the aforementioned binding buffer which is diluted to 4 to 5 times, though different kinds of buffers may be prepared separately. As a re-suspension buffer, water is suitable. As mentioned above, in the kit, the organic solvents such as chloroform and phenol which are used for nucleic acid extraction in the prior art and a chaotorope reagent, a surface active agent, and a bacteriolytic agent are not included.

### - Nucleic acid isolation instrument

In the method of the present invention for isolating a nucleic acid from a cell containing sample, using:
a magnet with a removable cover attached and
the following containers arranged on one table:
   (1) a container 1 for mixing a cell-containing sample and magnetic beads to which cells can be adhered,
   (2) a cleaning tank for cleaning the magnetic beads with cells attached, and
   (3) a container 2 for heating the cleaned magnetic beads,
the process of adhering and cleaning cells in the cell-containing sample to magnetic beads and isolating a nucleic acid from the cells is performed.

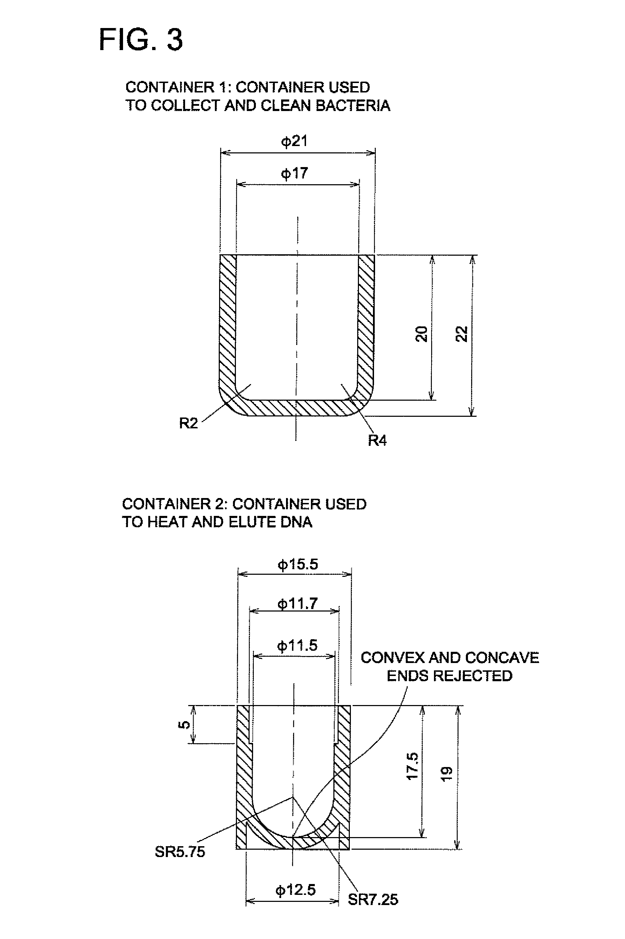

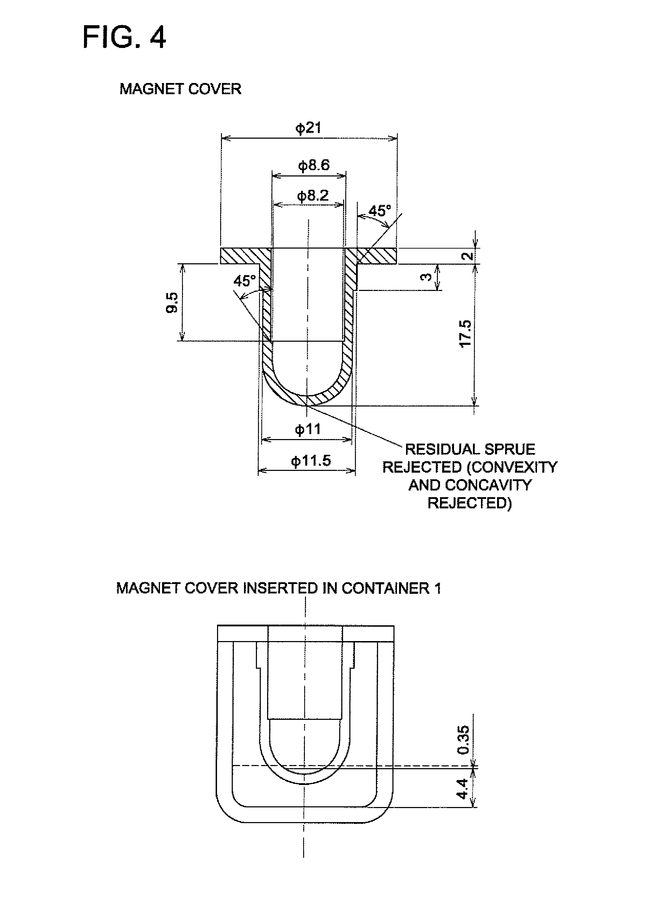

A schematic diagram of the suitable nucleic acid isolation instruments for executing the method of the present invention is shown in Figs. 3 and 4. Further, Fig. 2 shows an aspect of the nucleic acid isolation method using the magnetic support, showing the instruments for enabling solid-liquid separation by the magnetic process more briefly and quickly and the use method thereof.

The suitable instruments for executing the method of the present invention include at least a sample container (Container 1) having magnetic beads dispersed beforehand in a liquid (preferably a binding buffer) for collecting bacteria, a cleaning container (that is, a cleaning tank) including a cleaning buffer used for cleaning, a nucleic acid collection container (Container 2) including a re-suspension buffer used for heating and nucleic acid elusion, and a set of a magnet and a magnet cover. These containers are a cylindrical container for internally storing the set of magnet and magnet cover, which are characterized in that at least the internal bottom of the container used for heating and nucleic acid elusion and the internal bottom of the magnet cover are in a round shape.

The materials of the containers are glass or plastics and particularly, the resins such as polyethylene, polypropylene, polystyrene, and polycarbonate are used preferably. Examples of the concrete shape and size of the containers are shown in Fig. 3. In this case, two kinds of containers such as Container 1 used for bacteria collection and cleaning and Container 2 which is smaller than it and is used for heating and nucleic acid elusion are used.

Regarding Container 2, to collect efficiently a nucleic acid by reducing a loss thereof, the inside of the container is preferably in a round bottom shape. For example, in the bottom of Container 2 shown in Fig. 3, the inner wall and outer wall are rounded respectively at SR5.75 and SR7.25 in spherical shapes with radii of 5.75 mm and 7.25 mm. The bottom thereof, to speed up, if any, the heat conduction at time of heating, has a wall thinner than those of the other portions. Further, at the location at a distance of 5 mm from the upper opening, the step parts (corresponding to φ11.5 and φ11.7) are installed and when inserting the magnet and magnet cover, they are positioned. At that time, the re-suspension buffer (50 µL in Container 2 shown in Fig. 3) for extracting a nucleic acid positioned at the round bottom center of Container 2, to move conveniently in the neighborhood of the wall surface, has high thermal conductivity.

The drawn container is an example and the shape and size of the container may be selected properly as required. The buffer liquid to be put into the container is as described above.

Container 1, Container 2, and the cleaning tank which are described above are arranged desirably on one table, preferably one rectangular or circular table in the order in which they are processed.

Magnetic beads are used as a magnetic support. The magnetic beads are as described above, though the constitution of a preferable magnetic substance is iron oxide (Fe₃O₄) beads with a polymer coated. The magnetic beads, preferably magnetic beads with a size of 0.5 to 10 µm with a weight of 1 mg/mL in a dispersion liquid are put into a sample container beforehand. Therefore, labor of pouring a predetermined amount of a magnetic carrier into the sample container is saved. The magnetic beads have a property that they attract each other and are condensed, so that it is desirable generally to store them in a liquid. Even in the liquid, they are settled and condensed, though by slight vibration (for example, by stirring), they are dispersed in the liquid. Therefore, if the dispersion medium is neutral (pH 5 to 9), the composition thereof is no particular object and for example, water or 5M LiCl may be cited.

The magnet cover is a cover for covering the magnet during solid-liquid separation and on the surface thereof, the magnetic beads are adsorbed by the magnetic action of the magnet mounted internally. Therefore, to make the magnetic force of the magnet act effectively on the magnetic beads, they are desirably thin though depending on the intensity of the magnetic force of the magnet, for example, 0.25 to 2 mm, preferably 0.25 to 1.5 mm. The material is not restricted particularly, though the magnetic beads are disposable, thus they are preferably made of plastics. Concretely, polystyrene, polypropylene, and polyvinyl chloride may be cited. Among them, polypropylene is preferable particularly in the respect that it does not adsorb a nucleic acid. The shape of the magnet cover is no particular object, though the magnet cover is a form that it is sufficiently large at least to interrupt the magnet from the liquid in the aforementioned container and covers the magnet, so that it depends on the shape of the magnet. For example, it is cylindrical or columnar. Further, the cover may be a hard cover free of changing in the form or may be flexible in order to hold internally the magnet. When the cover is hard, the internal surface of the cover is desirably shaped so as to support the magnet and remove it easily. The size thereof, since the cover is inserted into the sample container aforementioned, must be smaller than the inside diameter of the container and furthermore, it is designed in consideration of the size of the magnet, the size of the container, and the liquid quantity of the container.

In Fig. 4, as a typical example of the magnet cover, the aspect of the round bottom tube shape is shown. The magnet inserted into the magnet cover is not inserted up to the bottom but is stopped halfway. In the drawing, so as to make the positioning of the lower limit (9.5 mm in the drawing) of insertion appropriate, a slight step part is installed on the inner wall of the magnet cover. Furthermore, also on the outer surface wall of the cover, a slight step part is preferably installed for convenience when positioning and fixing it. Container 1 and Container 2 shown in Fig. 3 and the magnet cover shown in Fig. 4 are designed under the condition that the magnet is in a columnar shape with a diameter of 8 mm, and the quantity of the sample liquid is 1 mL, and the quantity of the cleaning buffer is 1 mL, and the quantity of the re-suspension buffer is 50 µL. According to the quantity of each liquid, that is, so that the volume difference between Container 1 and the magnet cover becomes almost equivalent to the quantities of the sample liquid and cleaning buffer and the volume difference between Container 2 and the magnet cover becomes almost equivalent to the quantity of the re-suspension buffer, Container 1, Container 2, and the magnet cover are designed. When a sample liquid or a buffer liquid of 100 µL to 2 mL is put into the cleaning buffer, the magnet cover can be inserted into Container 1. Similarly, when a re-suspension buffer of 50 to 100 µL is put into Container 2, the magnet cover can be inserted into Container 2.

Further, the magnet, so as to be removed easily from the magnet cover, is desirably in an aspect that it is projected upward from the top of the magnet cover. The degree of the projection may be set properly as required.

A set of these instruments and the reagents aforementioned are merged as one set, thus an exclusive kit for the nucleic acid isolation method of the present invention is structured. Any of the instruments is structured generally as a consumption article for one analysis. It is desirable to sterilize beforehand the container including a buffer and prevent contamination by unwanted bacteria.

When executing the method of the present invention, the aforementioned consumption articles and also a mixer for stirring, a stirrer, a heater for heating, and a magnet used for solid-liquid separation by the magnetic process are used. Concretely, the stirring is desirably vibration due to a test tube mixer or fall-down mixture for rotating the container and as a heater, a manageable dry heat block for adjusting minutely the temperature is desirable. The magnet may be a bar magnet. The kind of magnet may be either of an electromagnet and a permanent magnet, though from the viewpoint of simplicity and operability, the permanent magnet is preferable and particularly from the viewpoint of the intensity of the magnetic force, a neodymium magnet is preferable.

These are generally apparatuses installed in the inspection chamber, and the apparatuses are used together with the kit, thus the method of the present invention can be executed. Further, whenever necessary, a part of such apparatuses may be included as a structure of the kit.

The preferable nucleic acid isolation method using the aforementioned kit is performed as indicated below.

It is preferable to set the quantity of the sample liquid to 100 µL to 5 mL, the quantity of the cleaning buffer to 250 µL to 1 mL or so which is equivalent to the quantity of the sample liquid, and the quantity of the re-suspension buffer to 5 to 100 µL.

A nucleic acid is separated by stirring and mixing magnetic beads for adhering cells and a sample liquid including cells in Container 1, adsorbing the concerned cells to the magnetic beads, then taking out the magnetic beads from Container 1, thereby separating them from the sample liquid, next cleaning them in the cleaning tank, then:
suspending the concerned magnetic beads in the re-suspension buffer in Container 2, and heating Container 2 at 80 to 120 °C for 20 to 300 seconds.

The aforementioned solid-liquid separation by magnetism is executed by the method for inserting the magnet with a cover attached into the container including the magnetic beads, then adhering the magnetic beads to the cover, thereby separating them from the liquid. The cell membranes of the cells adhered to the magnetic beads are destroyed by heating and the nucleic acid in the cells are dissolved into the re-suspension buffer simultaneously with the cell dissolution by heating. After heating as mentioned above, the magnetic beads are accumulated and taken out by the magnetic force and the taken-out magnetic beads may be abolished. The reason that the magnetic beads are taken out at this stage is that the concerned magnetic beads are prevented from being brought in the nucleic acid amplification reaction which will be executed later.

In this way, the nucleic acid dissolved in the re-suspension buffer remaining in Container 2 is separated and the nucleic acid liberated from the cells in the sample can be collected. Further, by the method of the present invention, the overall nucleic acid isolation process can be completed within 600 seconds.

It will be explained more in detail by referring to Fig. 2. (1) A proper amount of a cell-containing sample (for example, urine) is put into a sample container (Container 1 aforementioned) and is mixed with magnetic beads pre-put in the container by stirring them using a mixer. The cells (for example, bacteria) in the sample are adhered to the magnetic beads. (2) A magnet with a cover attached is inserted into the sample container aforementioned and the magnetic beads immersed in the sample liquid in the container are accumulated on the outer peripheral surface of the cover by the action of the magnet. By doing this, the magnetic beads are adhered to the cover and the solid-liquid separation from the sample can be enabled. (3) The magnet with a cover to which the magnet beads are adhered is pulled out from the sample container and is moved to the cleaning tank (that is, the cleaning buffer including container). (4) The magnet is removed from the cover and is pulled up from the cleaning tank. The magnetic beads adhered to the cover are removed from the cover, are re-dispersed in the cleaning buffer, and are cleaned by moving the cover up and down. (5) The magnet is inserted again into the magnet cover in the cleaning tank.

### (6) The magnetic beads are accumulated again on the outer surface of the magnet cover.

Similarly to (3), the magnetic beads with the magnet with the cover attached pulled out are moved to the nucleic acid collection container (Container 2 aforementioned).

Similarly to (4), the magnet with the cover removed is pulled up from the nucleic acid collection container and the magnetic beads are re-dispersed in the re-suspension buffer. The suspension liquid is heated and the cell membranes of the cells adhered to the magnetic beads are destroyed (or bacteriolysis is executed). Similarly to (5) The magnet is inserted into the magnet cover in the nucleic acid collection container and similarly to (6) the magnetic beads are accumulated. The magnet with the cover attached is pulled out and the magnet cover and magnetic beads are abolished.

### (7) The buffer is collected and the nucleic acid is obtained. In (7) shown in Fig. 2, a tube like a capillary for absorbing and collecting a liquid including a nucleic acid is inserted into the container.

By a series of operations aforementioned, when it is repeated to just mount the external magnet in the magnet cover thereof and then remove it, the solid-liquid separation, movement, and cleaning can be executed easily. Further, the concerned magnet does not touch directly the liquid in the container, so that in analysis of many samples, the same magnet may be mounted on the respective magnet covers. Therefore, the magnet itself can be shared, so that basically, only one magnet may be prepared.

### Nucleic acid isolation apparatus

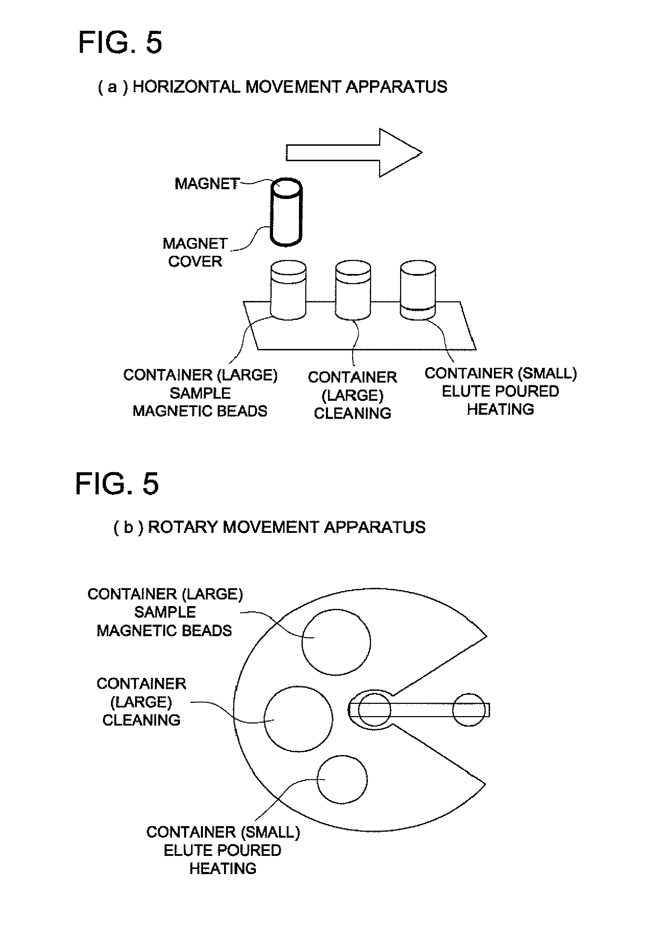

The operation for the nucleic acid isolation apparatus using the instruments aforementioned is shown schematically in Figs. 5(a) and 5(b). In the horizontal movement apparatus shown in Fig. 5(a), when the magnet and magnet cover move horizontally and move up and down so as to get in and out from a predetermined container, the cell processing in the sample is performed sequentially. In the horizontal direction in which the magnet and magnet cover move, on one table (preferably a rectangular table), (1) Container 1 for mixing a cell-containing sample with magnetic beads for adhering cells, (2) a cleaning tank for cleaning the magnetic beads with cells adhered, and (3) Container 2 for heating the magnetic beads after cleaning are arranged in the order in which they are processed.

Further, the drive section for moving the magnet and magnet cover horizontally and vertically is not shown in Fig. 5(a). Further, a mechanism for moving simultaneously the magnet and magnet cover or moving only the magnet when necessary may be included. Particularly, when heating cells, at least the magnet, to avoid a magnetic loss due to heat, must be withdrawn from a container for holding cells.

The rotary movement apparatus shown in Fig. 5(b) is a rotary system apparatus instead of moving horizontally as shown in Fig. 5(a). By rotation of the central rotary shaft, the magnet and magnet cover which are connected thereto move around and move up and down so as to get in and out from the predetermined container. In the rotational direction in which the magnet and magnet cover move, on one table (preferably a circular table), Container 1, the cleaning tank, and Container 2 which are mentioned above are arranged in the order in which they are processed.

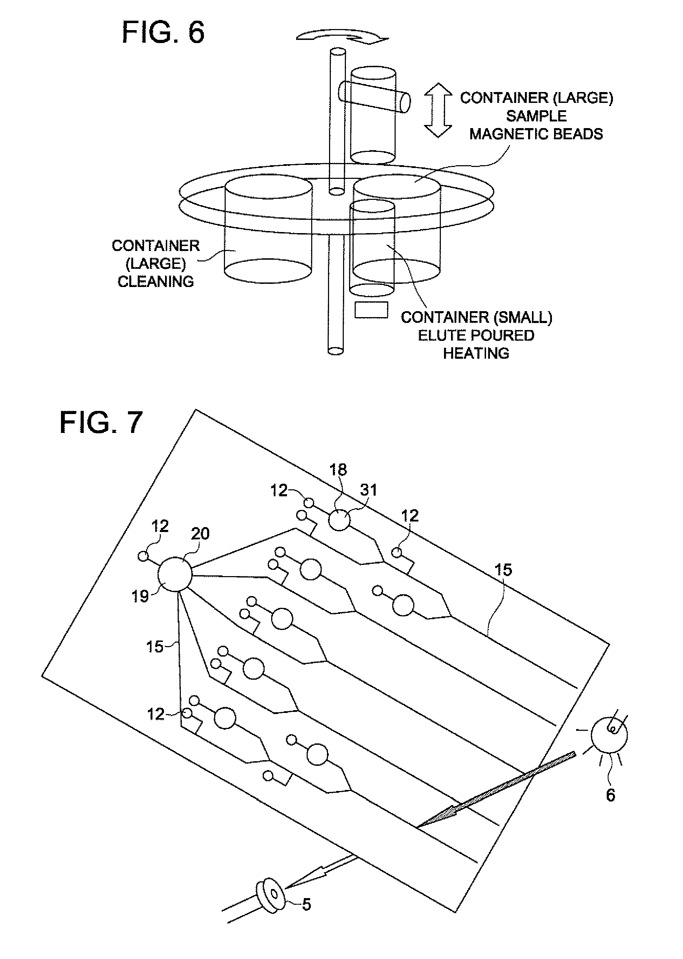

Fig. 6 is a drawing showing movement of each section of the rotary movement apparatus so as to be seen clearly. The drive section of the apparatus for moving it horizontally and vertically is omitted in the drawing. Also in this apparatus, a mechanism for moving simultaneously the magnet and magnet cover or moving only the magnet when necessary may be included. Particularly, when heating cells, at least the magnet, to avoid a magnetic loss due to heat, must be withdrawn from a container for holding cells.

Further, including this case, the nucleic acid isolation apparatuses shown in Figs. 5(a) and 5(b) use respectively the solid-liquid separation by the magnetic action and also in Fig. 5(b), the action of the centrifugal force is not used.

The nucleic acid isolation apparatus for executing the method of the present invention is composed of the nucleic acid isolation instruments such as a container, drive section for moving horizontally and vertically the magnet and magnet cover, a controller for controlling the movement thereof, and a temperature controller for controlling the temperature and when necessary, they are united. As an example of a preferable aspect, when a cell-containing sample liquid is poured into a sample container (Container 1) in which magnetic beads and a binding buffer are charged beforehand, a mechanical connection for moving the magnet and magnet cover and when necessary, an electric connection for control are made, thus the apparatus enters the operation state. In succession, the steps of mixing and stirring samples and buffers, heating, and separating a liberated nucleic acid are executed automatically as a series of continuous steps. In this nucleic acid isolation apparatus, the preset conditions of the execution procedure of the steps and timing are incorporated into the software loaded in the nucleic acid isolation apparatus as a program together with the movement mechanism of the magnet and magnet cover and temperature control, and the steps progress smoothly, and the target sample process is desirably controlled by a computer so as to be executed.

Figs. 2 to 6 are illustrations for explaining the instruments and apparatuses of the present invention and the shape, arrangement, and size of the parts shown in the drawing are strictly just an example. Therefore, for the whole or a part of the nucleic acid isolation apparatus and kit contents of the present invention, the structure, constitution, arrangement, shape and form, size, material, system, and method may be modified variously within a range which is not deviated from the object of the present invention.

### Gene inspection method

The gene inspection method of the present invention is a method including a stage of isolating a nucleic acid by the aforementioned method, amplifying the nucleic acid (gene) by an apparatus having a microchip, and detecting it.

As a nucleic acid analytical apparatus for executing the gene inspection method of the present invention, a microchip form may be included, thus a high-throughput analysis can be made.

### - Nucleic acid analytical apparatus

The nucleic acid analytical apparatus for executing the gene inspection method of the present invention is composed of an apparatus body in which a micropump, a controller for controlling the micropump, and a temperature controller for controlling the temperature are united and a microchip for nucleic acid amplification and detection mountable on the apparatus body. When a specimen liquid is poured into a specimen receiving section of the microchip in which a reagent is charged beforehand, and the microchip is mounted on the main unit of the nucleic acid analytical apparatus, a mechanical connection for operating the liquid feed pump and when necessary, an electrical connection for control are made. When the main unit and microchip are joined, the flow path of the microchip enters the operation state. Therefore, in an example of a preferable aspect, when the operation is started, feeding and mixing of the specimen and reagent and amplification and detection of a nucleic acid are executed automatically as a series of continuous steps.

The units taking charge of the control systems relating to each control of liquid feed, mixture, and temperature compose the main unit of the nucleic acid analytical apparatus of the present invention together with the micropump. The main unit of the apparatus, when the microchip aforementioned is mounted on it, can be used commonly for specimens. The aforementioned steps of liquid mixture, liquid feed, nucleic acid amplification, and detection, as preset conditions of the liquid feed procedure, capacity, and timing, are incorporated in the software loaded on the nucleic acid analytical apparatus as a program together with control for the micropump and temperature. According to the present invention, only the removable microchip aforementioned may be exchanged. The nucleic acid analytical apparatus of the present invention, since every component is miniaturized and is formed in a handy form, is not restricted on the location and time in use and the workability and operability are satisfactory. Many micropump units used for liquid feed are incorporated in the main unit of the apparatus, so that the microchip can be used as a disposable type.

### - Microchip for nucleic acid amplification and detection, micropump, and pump connection

As an example of a preferable aspect of the microchip for nucleic acid amplification and detection, the embodiment shown in Fig. 7 will be explained. For a specimen receiving section 20 and a reagent storing section 18, a micropump for feeding inner liquids of these storing sections is installed. The micropump is connected to the upper stream side of the reagent storing section 18 via a pump connection 12, and a drive liquid is fed to the reagent storing section by the micropump, thus the reagent is pressed out to the flow path and is fed. The micropump units are incorporated in the main unit of a separate nucleic acid analytical apparatus from the microchip for nucleic acid amplification and detection and when the microchip is mounted in the main unit of the nucleic acid analytical apparatus, it is connected to the microchip from the pump connection 12.

In this embodiment, as a micropump, a piezo-electric pump is used. Namely, the piezo-electric pump is composed of:
a first flow path in which the flow path resistance is changed according to the differential pressure,
a second flow path in which the rate of change in the flow path resistance to change in the differential pressure is smaller than that of the first flow path,
a pressure chamber connected to the first flow path and second flow path, and
an actuator for changing the inner pressure of the pressure chamber.

The details thereof are described in Japanese Patent Application 2001-322099 and Japanese Patent Application 2004-108285.

Regarding the nucleic acid amplification and detection chip used for the nucleic acid analytical apparatus aforementioned, an example of a preferable aspect will be described below. The microchip of the aspect includes at least the specimen liquid receiving section 20, reagent storing section 18, waste liquid storing section, micropump connection 12, and fine flow paths 15 and interconnects the sections with the fine flow paths. The microchip is specialized to permit a specimen liquid (a liquid containing an isolated nucleic acid) 19 to flow in the flow path for composing the nucleic acid amplification position installed on the downstream side of the specimen receiving section and then the flow path composing the position for detecting the amplified nucleic acid, mix it with a reagent 31 of the reagent storing section 18, thereby analyze the nucleic acid, and move and lock a waste liquid generated consequently to the waste liquid storing section. Furthermore, in addition to the storing sections, flow paths, and pump connection, the elements such as the liquid feed controller, reverse flow preventive section, reagent determining section, and mixing section are installed at functionally suitable positions by fine processing techniques.

Next, an example of a preferable aspect of the microchip will be indicated. The microchip for nucleic acid amplification and detection is a microchip prepared by combining properly two or more members of plastic resin, glass, silicon, and ceramics. The length and width thereof are generally several tens mm and the height is several mm. Preferably, the fine flow path and body of the microchip are formed by plastic resin which is processed and molded easily and cheaply and can be destroyed by fire and abolished easily. Particularly, the resins such as polyolefin, for example, polypropylene and polystyrene are excellent in moldability, so that they are desirable. The fine flow path, by the fine processing technique, is formed with a width and a height of about 10 µm to several hundreds µm, for example, a width of about 100 µm and a depth of about 100 µm.

### - Amplification and detection of nucleic acid

The isolated nucleic acid is amplified at the nucleic acid amplification position of the microchip for nucleic acid amplification and detection, and then the amplified nucleic acid is sent to the detection position of the microchip, thus the nucleic acid (gene) is detected. The nucleic acid is amplified by the DNA amplification method of PCR, SDA, LCR, ICAN, LAMP, TAM, TAS, 3SR, or NASBA. The amplified nucleic acid is analyzed by the regular method, for example, the hybridization method or gold colloid adsorption method.

For the whole or a part of the microchip and nucleic acid analytical apparatus, the structure, constitution, arrangement, shape and form, size, material, system, and method may be modified variously within a range which is not deviated from the object of the present invention.

### Embodiments

The apparatus names used in the following embodiments, concentration of used materials, use quantities, processing time, numerical conditions such as processing temperature, and processing methods are only preferred examples within the scope of the invention. Further, the present invention is not limited to these embodiments.

### Embodiment 1

100 mL of urine decided as positive chlamydia by Roche's Cobas Amplicor STD-1 is poured into the sample container shown in Fig. 3. In the sample container, 10 mg/mL of magnetic beads (chlamCAP beads) of BUGS'n Version U by Genpoint AS Ltd., 30 µL of a dispersion liquid, and 200 µL of a binding buffer are poured beforehand. The magnet cover is inserted into the sample container and the cover is rotated sideways for stirring to mix the sample liquid. The magnet is inserted into the cover in the sample container and is immersed into the sample liquid in the sample container, thus the magnetic beads are accumulated on the outer peripheral surface of the cover by the action of the magnet. The magnet with the cover attached to which the magnetic beads are adhered is withdrawn from the sample container and is transferred to the cleaning buffer containing container. The magnet is removed from the cover and is pulled up from the cleaning tank, and the magnetic beads are re-dispersed in the cleaning buffer, and the cover is rotated sideways, and the magnetic beads are cleaned. The magnet is inserted into the cover in the cleaning tank and the magnetic beads are accumulated on the outer peripheral surface of the cover. The magnet with the cover attached is pulled out from the cleaning tank and is transferred to the nucleic acid collection container containing a re-suspension buffer (50 µL of water). The magnet is removed from the cover and is pulled up from the nucleic acid collection container and the magnetic beads are re-dispersed in the re-suspension buffer. The nucleic acid collection container is heated at 100 °C for 3 to 5 minutes. The magnet is inserted again into the cover in the nucleic acid collection container, and the magnetic beads are accumulated on the outer peripheral surface of the cover, and the magnet with the cover attached is pulled out, and the magnet cover and magnetic beads are abolished. For the top clear part of the nucleic acid collection container, using LA Taq by Takara Biochemstry Ltd., under the conditions of at 95 °C for 5 minutes (at 95 °C for 30 seconds, at 60 °C for 50 seconds, at 72 °C for 35 seconds) with 40 cycles and at 72 °C for 5 minutes, the PCR amplification reaction is performed. The PCR primer is prepared and used on the basis of the base arrangement described in GenBank accession number X06707. The base arrangement is as indicated at the arrangement Nos. 1 and 2 of the arrangement table.

As a result of the electrophoresis, the target amplified fragment of 208 bp is confirmed. From the aforementioned protocol, the positive reaction by PCR is confirmed.

### Embodiment 1

100 mL of urine decided similarly as positive chlamydia, 5 mg/mL of BioMag streptavidin magnetic beads (BioMag streptavidin particles) by Polyscience Ltd., 30 µL of a dispersion liquid, and 200 µL of a binding buffer (isopropanol, 0.75M ammonium acetate NH₄Ac) are stirred and mixed. After execution of incubation at normal temperature for 1 to 5 minutes, the magnetic beads are separated by centrifugation of 2000 G for 1 minute or a magnet and the top clear part is abolished. 250 µL of a 4.5 times diluted binding buffer (that is, a solution that a binding buffer is diluted at 4.5 times in sterilized water) is added and stirred as a cleaning buffer, thus the magnetic beads are cleaned. The magnetic beads are separated by centrifugation of 2000 g for one minute or a magnet and the top clear part is abolished. 100 µL of sterilized water is added and the magnetic beads are heated at 94 °C for one minute. The top clear part, under the condition of 58 °C and 60 minutes, is detected by the ICAN (isothermal chimera primer initiated nucleic acid amplification, registered trademark) amplification reaction and the solid layer plate light emission method. The chimera oligonucletide primer by ICAN method and light emission detection probe are prepared according to Embodiment 6 of Japanese Patent Application Laid-Open Announcement 02/052043 (W02002/052043). By the protocol aforementioned, the positive reaction is confirmed.

### Embodiment 3

100 mL of urine decided similarly as positive chlamydia, 10 mg/mL of magnetic beads (chlamCAP Beads) of BUGS'n Version U by Genpoint AS Ltd., 30 µL of a dispersion liquid, and 200 µL of a binding buffer are stirred and mixed. Hereafter, up to the amplification and detection, the mixed solution is processed similarly to Embodiment 2. Namely, the incubation at normal temperature, separation by centrifugation or a magnet, abolishment of the top clear part, stirring and cleaning by a 4.5 times diluted binding buffer, separation by centrifugation or a magnet, abolishment of the top clear part, addition of sterilized water, heating, ICAN, and detection are executed sequentially and the positive reaction is confirmed.

### Embodiment 4

To 100 µL of urine decided as negative chlamydia by Roche's Cobas Amplicor STD-1, 25 copies of control plasmid having the same base arrangement as that of the amplification target portion is added and is used as a sample. By the same protocol as that of Embodiment 3, the sample processing, amplification, and detection are executed and the positive reaction is confirmed.

The present invention does not require use of severe chemicals for a sample containing cells, destroys the cells quickly and effectively, thereby can isolate simply a nucleic acid.

When moving magnetic beads, the present invention operates the magnetic beads by inserting or pulling out the magnet with a removable cover attached, thus the solid-liquid operation is simple and at the nucleic acid isolation process, samples are lost little.

When detecting genes of microbes of fungi (bacteria, true fungi) or yeast, if the concentration of the objective bacteria in a sample is very low, it is impossible practically to trap it, while the method of the present invention is condensed to bind cells to the magnetic support, thereby can insolate a nucleic acid with high purity.

The method of the present invention can be executed quickly and simply with simple reagents, components, and instruments, so that it can be kitted easily, and the solid-liquid separation mechanism is mechanized and automated, thus a nucleic acid isolation apparatus can be realized, and furthermore, a nucleic acid can be isolated efficiently.

The containers of the nucleic acid isolation instruments of the present invention and magnet cover are shaped so as to execute quickly solid-liquid separation by magnetism and are disposable, thus they can respond to many sample processes.

## Claims

1. A method for isolating a nucleic acid from a cell-containing sample by using a magnet with a removable cover attached thereto and a plurality of containers arranged on a table, the plurality of containers comprising (1) a first container for mixing a cell-containing sample and magnetic beads to which cells can be adhered, (2) a cleaning tank for cleaning the magnetic beads with cells adhering thereto, and (3) a second container for heating the cleaned magnetic beads, the method comprises:
mixing a liquid including cell-containing sample and magnetic beads in the cell-containing sample in the first container so as to adhere cells in the cell-containing sample to magnetic beads;
taking out the magnetic beads with cells adhering thereto from the first container and cleaning the magnetic beads; and
suspending the magnetic beads in a re-suspension buffer in the second container and heating the second container at 80 to 120 °C for 20 to 300 seconds so as to isolate a nucleic acid from the cells, wherein
all the processes are completed within 600 seconds.

2. The method of claim 1, further comprising suspending the magnetic beads at least in a stage selected among before mixing the magnetic beads with the liquid, after mixing the beads with the liquid, and during cleaning the magnetic beads.

3. The method of claim 1, wherein the cells of said cell-containing sample is a microbe belonging to one of the Chlamydia, Neisseria gonorrhea or Mycobacterium.

4. The method of claim 1, wherein the containers and cleaning tank are arranged in the order in which they are processed on a rectangular or circular table.

5. A method of identifying cell species comprising: identifying a nucleic acid obtained by the isolating method of claim 1 by a nucleic acid amplification method.

6. A kit for implementing the method of claim 1, comprising a plurality of containers in which magnetic beads, a cleaning buffer and a re-suspension buffer are respectively charged beforehand.

7. An apparatus for implementing the isolating method of claim 1.

8. A gene inspection method comprising: isolating a nucleic acid from a cell-containing sample by the isolating method of claim 1 and amplifying and detecting the nucleic acid by an apparatus having a microchip.
